Europäisches Patentamt

European Patent Office

Office européen des brevets

(11) Publication number: **0 451 280 A1**

# EUROPEAN PATENT APPLICATION
## published in accordance with Art. 158(3) EPC

(21) Application number: 90912890.2

(22) Date of filing: 20.02.90

(86) International application number:
PCT/SU90/00046

(87) International publication number:
WO 91/03444 (21.03.91 91/07)

(51) Int. Cl.5: **C07C 4/04**, C10G 9/00,
C10G 9/20, C10G 9/38

(30) Priority: 04.09.89 SU 4730941

(43) Date of publication of application:
16.10.91 Bulletin 91/42

(84) Designated Contracting States:
DE DK FR GB IT LU NL SE

(71) Applicant: **KAZAKHSKY MEZHOTRASLEVOI NAUCHNO-TEKHNICHESKY TSENTR SVS**
**ul. Kirova, 172**
**Alma-Ata, 480012(SU)**

(72) Inventor: **XANDOPULO, Georgy Ivanovich**
**ul. Mira, 166-34**
**Alma-Ata, 480064(SU)**
Inventor: **LEIMAN, Zoya Alexeevna**
**mikroraion Kazakhfilm, 24-31**
**Alma-Ata, 480117(SU)**
Inventor: **TOMAROVSKAYA, Natalya Petrovna**
**pr. Lenina, 36-83**
**Alma-Ata, 480100(SU)**
Inventor: **VORONOV, Nikolai Jurievich**
**ul. Isaeva, 161-1**
**Alma-Ata, 480008(SU)**

(74) Representative: **Ablewhite, Alan James et al**
**MARKS & CLERK 57/60 Lincoln's Inn Fields**
**London WC2A 3LS(GB)**

(54) METHOD AND DEVICE FOR OBTAINING LOWER OLEFINES.

(57) The method provides for heating a hydrocarbon raw material, its pyrolysis at a temperature of 600-800 °C in the presence of intermediate products of hydrogen and oxygen combustion which are produced in the flame torch and are fed along the axis and/or along the periphery of the raw material flow. The time of reaction between the raw material and addition agents at a temperature of 600-650 °C is 1-30 msec. The device comprises a casing (1) with a heating and a pyrolysis zone (4, 5). In the zone (5) is mounted a burner (8) whose casing (9) is provided with a nozzle (10) mounted in its end-face (11) and with nozzles (12) mounted along its periphery. The zone is provided with a pipe (13) with branches (14) for feeding the oxidizer and a channel (15) for feeding the fuel, interconnected to each other.

The present invention relates to the art of processing petroleum crudes and, more particularly, to a method and apparatus for producing lower olefines.

To improve selectivity of a process of pyrolysis and to increase the yield of olefines, ethylene in particular, it is necessary to elevate the process temperature and to reduce the time of contact of a hydrocarbon feedstock with a heat-transfer agent, i.e. to make the pyrolysis conditions more severe, or to introduce initiating additives into a hydrocarbon feedstock which are capable to easily decompose into radicals at temperatures below that of the pyrolysis process /halogenated hydrocarbons, organic and inorganic peroxides, sulfur-containing compounds, and the like/.

Widely known in the art is a process of oxidizing pyrolysis of a hydrocarbon feedstock, wherein the heat necessary for conversion of hydrocarbons is obtained as a result of combustion of a a portion of a starting feedstock in the atmosphere of oxygen directly in the zone of pyrolysis. This process is intended mainly for production of acetylene. In the production of olefines the oxidizing pyrolysis has not found wide application due to process troubles at certain stages, low technical and economic indices /the yield of olefines did not exceed 10% by weight/, a complicated procedure of recovery of the desired products due to the formation of a considerable amount of by-products of combustion /CO, $CO_2$ in the amount of 40 to 45% by volume/ contaminating the gas resulting from the pyrolysis process. Furthermore, the process is hardly amenable to control.

Known in the art is a method and an apparatus for production of lower olefines by high-temperature pyrolysis of a hydrocarbon feedstock in presence of a gaseous heat-transfer agent /US, A, 4,256,565/.

The apparatus contains a high-pressure vessel wherein a reactor intended for pyrolysis of a feedstock is mounted with a possibility of performing translatory motion, Such a structure of the reactor makes it possible to maintain the pressures inside and outside thereof equal, which permits the operation of said reactor at high temperaturesand high pressures. The reactor is equipped with a means for supplying of a hydrocarbon feedstock and discharging of the pyrolysis process products, and pipe-lines for introducing of oxygen and preheated hydrogen thereinto. The reactor includes two reaction zones communicating and spaced apart at a distance of 5 to 12 times the diameter of the first reaction zone.

Mounted in the discharge section of the second zone is a homoentropic Laval nozzle, positioned in the direction of a pyrolyzed feedstock movement and intended for prompt cooling of the pyrolysis process products, A means for introduction of a cooling agent /water for instance/ is also located in this discharge section, The combined aerodynamic and jet cooling makes it possible to lower the temperature to a specified value within 1 to 2 ms.

The method is implemented with the disclosed apparatus as follows. A flow of gaseous oxygen is introduced into the central part of the first reaction zone at a rate of 300 to 600 volumes per a volume of a feedstock being pyrolyzed and along the periphery of this flow of oxygen in the same direction a parallel flow of preheated hydrogen is introduced at a rate of 1,000 to 2,000 volumes per a volume of a starting feedstock subjected to pyrolysis. In the first reaction zone hydrogen spontaneously reacts with oxygen with the formation of a flow of a heat-transfer medium having a temperature of 1,000 to 2,000°C containing mainly hydrogen and an inconsiderable amount of steam. This flow is introduced into the second reaction zone where it meets one or more flows of a preheated hydrocarbon feedstock also fed into the second zone. The speeds and rates of the flows are adjusted so that excellent mixing and required super-quick heating of a hydrocarbon feedstock takes place providing for its pyrolysis under a temperature of 1200 to 1800°C within 1 to 10 ms. Soon after the pyrolysis products cool down.

The process allows the pyrolysis of different kinds of starting hydrocarbon feedstock ranging from ethane to gasoil as well as aromatic hydrocarbons. However, practical implementation of this process is economically inefficient, since, first of all, high quantities of aeficit hydrogen are required /or the order of 1,000 to 2,000 volumes per a volume of a feedstock being subjected to pyrolysis/, as well as high quantities of oxygen /300 to 600 volumes per a volume of a starting feedstock/, and secondly, due to contamination of the pyrolysis products with carbon oxides.

Furthermore, the disclosed apparatus does not provide for the maximum yield of specified final products of a hydrocarbon feedstock pyrolysis, since the heating agent production zone /the first reaction zone/ and a hydrocarbon feedstock pyrolysis zone /the second reaction zone/ are spaced apart in the reactor vessel at a predetermined distance, resulting in that a part of the energy of heating and a part of the free radicals transferred from the flame torch to the feed stock are lost.

Also known in the art are a process and an apparatus for producing lower olefines (SU, A, 1,268,558). The apparatus comprises a shell with a means for supplying of a hydrocarbon feedstock and discharging of pyrolysis products, said shell incorporating a heating zone and a pyrolysis zone consecutively located in the direction of a feedstock movement, and a burner concentrically located therein and comprising a cylindric casing with a nozzle positioned coaxially therein at an end thereof, and a central conduit for supplying of an oxidizer located in the cylindric casing and forming, with the internal surface of the burner casing wall, an annular passage for feeding a fuel to the nozzle. The burner is located in the heating zone.

The process includes the steps of heating of a flow of a starting hydrocarbon feedstock in the heating zone to a temperature of 200 to 300°C and introducing of initiating additives therein at this temperature, said additives being intermediate products of combustion of a fuel - hydrocarbons in an oxidizing agent - oxygen, formed in the flame torch within the burner nozzle. Initiated in this way hydrocarbon feedstock passes further to the pyrolysis zone where a temperature of 750 to 920°C is maintained. The pyrolysis products are cooled and the specified final compounds are separated therefrom. To generate a flame torch an oxidizer is supplied in an amount of 0.1 to 0.4 1, and a hydrocarbon fuel is supplied in an amount of 0.03 to 0.08 1 per litre of the starting feedstock.

The yield of lower olefines in this method is within the range of 29 to 47 mass %, the maximum level being achieved at the temperature of 920°C. However, to maintain such a high temperature of a pyrolysis process is disadvantageous due to more active development of secondary reactions resulting in decomposition of the specified final products, which deteriorates stability of the yield level thus resulting in considerable reduction of the apparatus operational life time.

In the disclosed apparatus the burner is located in the zone of heating of a pyrolyzed feedstock. Such an arrangement of the burner in the zone of a minimal temperature /200 to 300°C/ brings about reduction of the rate of reactions between radicals and a hydrocarbon feedstock. The life time of radicals at such temperatures is rather short, while the newly formed chains of pyrolysis disappear having not reached the pyrolysis zone due to the disadvantageous temperature conditions for the chains propagation.

Thus, the known process features a relatively low yield of lower olefines, ethylene in particular, which amounts to 29 to 35% by mass. This effect is obviously associated with a low temperature of a starting feedstock when initiated, as well as considerable distance between the main zone of pyrolysis and the burner flame torch.

The use of initiating additives from a single flame torch is inefficient due to a small surface area of contact of active particles with a starting hydrocarbon feedstock subjected to pyrolysis, which adversely affects the yield of the specified final products.

Furthermore, the pyrolysis products are heavily contaminated with carbon oxides which complicate the recovery of the specified final products from the pyrolysate.

This invention is based on the problem of providing a method for producing of lower olefines by way of selecting appropriate initiating conditions and changing the quality of initiating additives and an apparatus for carrying out said method with a modified construction arrangement of the burner and its location, which method and apparatus ensure an enhanced yield of the specified final products featuring improved quality.

This problem is solved by that in a disclosed method for producing of lower olefines including the steps of heating a hydrocarbon feedstock, its pyrolysis in the presence of initiating additives - intermediate products of burning of a fuel and an oxidizer formed in a flame torch, cooling of the pyrolysis process products followed by isolation of specified final products therefrom, according to the invention, the intermediate products of burning of hydrogen used as a fuel, and oxygen, which is used as an oxydizer, are used as initiating additives produced in a flame torch, which additives are introduced along the axis and/or periphery of the hydrocarbon feedstock flow in a pyrolysis zone by way of appropriately positioning therein of at least one additional flame torch to ensure reaction time of said initiating additives and said hydrocarbon feedstock being eaual 1 to 30 ms at a temperature within the range of 600 to 650°C, while the pyrolysis temperature is 600 to 800°C.

Implementation of the process according to the present invention under the above-specified conditions based on the use of qualitatively novel initiating additives makes it possible:
- to attain a yield of olefines, ethylene in particular, equal to 30 to 52% by mass which, as calculated for the maximum yield relative to a thermal pyrolysis, is increased by 5 to 10% by mass;
- to reduce the pyrolysis temperature by 60 to 110°C which results in reduction of the termal strain, prolongs the furnace overhaul period and over-all operational life time;
- to conduct the process using the existing equipment;
- to ensure such a rate of pyrolysis which results in a higher productivity of a furnace;
- to avoid any possibility of the formation of carbon oxides, to reduce resin and coke formation;
- to use different kinds of feedstock for pyrolysis, ranging from ethane to vacuum gasoils;
- to reduce consumption of diluting stream due to the formation of additional stream in the hydrogen-oxygen torch.

The choice of intermediate combustion products formed in the hydrogen-oxygen flame for initiation is effected according to the following considerations.

In the process of burning of hydrogen in oxygen, as distinct from a hydrocarbon flame, carbon oxides are not formed, but radicals H, OH, and $HO_2$ originate, which are more effective for initiation than hydrocarbon radicals. The effect of the torch on the process of pyrolysis is expressed in the combined form of total influence of active particles of the torch simultaneously with a thermal overheating of the starting feedstock in the torch zone. Therefore, the temperature of the hydrogen-oxygen flame, exceeding the temperature of hydrocarbon flame by 500 to 600°C, also contributes to the effect of initiation.

To improve the yield of olefines it is advantageous carry out pyrolysis process with the contact time of from 0.05 to 1.5 seconds.

It is expedient that the flame torch be shaped by supplying a flow of oxygen and, along its periphery, by a parallel flow of hydrogen in a volume ratio of 1:/2 to 10/ and at a rate of 0.1 to 1.0 l of hydrogen and 0.01 to 0.5 l of oxygen per 1 litre of a starting feedstock.

This makes it possible to confine oxygen within the torch zone and avoid its penetration into a starting feedstock and oxidation thereof to carbon oxides. Furthermore, this enables carrying out of the process of initiation under optimal conditions.

To ensure most effective use of the initiating additives they should be introduced along the axis and/or the periphery of the flow of a starting hydrocarbon feedstock.

The hydrocarbon starting feedstock heated to a temperature of 600°C is fed into the zone of hydrogen-oxygen flame torches, wherein it is subjected during 1 to 30 ms to an active effect of intermediate products of combustion, i.e. radicals H, OH, and $H_2O$ on the one hand, and to a local effect of a thermal shock of the flame, on the other.

In this case the starting hydrocarbon feedstock is decomposed and chains of pyrolysis appear at relatively low temperatures of said starting feedstock within the temperature range of 600 to 650°C. This results in a reduction of the pyrolysis temperature by 60° to 110°C accompanied by a general reduction of the thermal tension of operational conditions of the apparatus.

Owing to the arrangement and to the appropriate orientation of several torches in the pyrolysis zone, it is possible to increase the surface area of interaction of a starting feedstock with said active particles, thus increasing the efficiency of initiation of pyrolysis and hence the yield of olefines. The required interaction time of 1 to 30 ms has been found experimentally from the predetermined number of torches of hydrogen-oxygen flame and the length of the initiation zone defined by the temperatures between 600 and 650°C.

This temperature range is determined by the maximum efficiency of initiation, since at a temperature below 600°C pyrolysis of hydrocarbons is impossible, whereas at a temperature above 650°C, a complete alteration of the process parameters is required.

Pyrolysis of a hydrocarbon feedstock, when initiated with a hydrogen-oxygen flame, starts at the temperature of 600°C and ends at the temperature of 800°C, the greatest amount of olefines being formed at a temperature interval of from 700 to 800°C.

At temperatures below 800°C the starting feedstock is fully converted thus reducing the energy consumption. The pyrolysis gaseous products are further discharged from an the apparatus, cooled down and separated.

The problem is also solved by providing an apparatus for carrying out the disclosed method and comprising a shell with means for supplying therein of a hydrocarbon starting feedstock and for discharging pyrolysis products therefrom and having a heating zone and a pyrolysis zone arranged consecutively therein along the direction of the flow of the feedstock being processed, a burner mounted in the shell coaxially thereto and having a cylindrical casing with a nozzle mounted coaxially therewith at an end-face thereof, and a central conduit arranged in the cylindrical casing and adapted for feeding an oxidizing agent, said conduit defining with the inner wall of the burner casing an annular passage adapted to feed a fuel to the nozzle, wherein, according to the invention, the burner is positioned in the pyrolysis zone, particularly in that part thereof where the temperature is in the range of 600 to 650°C, and includes at least one row of auxiliary nozzles equidistantly arranged around the periphery of its casing, the central conduit being provided with a number of branches corresponding to the number of the auxiliary nozzles and having their outlets communicating with the fuel feeding passage, each branch being positioned coaxially with the respective nozzle.

Such a construction arrangement of the burner and its location ensure more effective distribution of the heating energy and the initiating radicals within the volume of a feeded hydrocarbon feedstock due to the increased contact area of the feedstock with the flame torches created by the burner auxiliary nozzles. Overheating of the steam-feedstock mixture in the zone where the flame torches are localized is precluded, thereby eliminating formation of by-products /CO, $CO_2$, soot, acetylene/ reducing the yield of olefines. The flame torches generated by the auxiliary nozzles, providing the latter are arranged appropriately, merge into one integrated flame torch which is characterized by stability in conditions featuring high linear speeds of the feedstock movement in the apparatus shell, whereby the initiation process efficiency is enhanced.

Advantageously, the apparatus includes additionally a burner arrangement assembled on the shell outer surface in the vicinity of the burner, comprising at least one row of nozzles fixed in the wall of the shell and evenly distributed over the perimeter thereof, the outlet ends of said nozzles protruding beyond the wall inner surface, an annular manifold for feeding a fuel fixed to the shell and communicating with the inlets of the nozzles, and the other annular manifold for feeding an oxidizer mounted on the outer wall of the first annular manifold and communicating with the inlets of the nozzles by pipe connections passing through the first manifold.

The distance between the burner and the nozzles of the burner arrangement is so selected as to preclude any interaction between the flame torches of the burner nozzles and of the burner arrangement nozzles.

The aforedescribed embodiment of the apparatus is most advantageously used in shells of a large cross-section, and facilitates the contact of the hydrocarbon feedstock with the flame torches over the whole area of the shell cross-section, thus ensuring the maximum yield of the specified final products.

It is expedient that the auxiliary nozzles of the burner and the nozzles of the burner arrangement be positioned with respect to the longitudal axis of the shell at an angle less than $90°$. This provides stability of burning in the regimes of high linear speed of movement of a hydrocarbon feedstock as well as uniform effect of heating energy and initiating additives thereon.

It is preferred that the burner is equipped with two rows of auxiliary nozzles, and the burner arrangement, with two rows of auxiliary nozzles, the nozzles of the preceeding row and the nozzles of the succeeding row being offset with respect to each other around the circumference and arranged in a staggered order.

This arrangement of the nozzles precludes formation of local overheating zones due to intersection of flame torches and ensure optimum contact time of the hydrocarbon feedstock with the initiating additives.

The invention will become more apparent from the description of specific exemplary embodiments thereof with references to accompanying drawings wherein:

FIG. 1 is a general view of the apparatus for producing of lower olefines.

FIG. 2 is a longitudal cross-section of a part A depicted in FIG. 1 with the burner coaxially positioned in the shell, an enlarged view.

FIG. 3 shows the same part A depicted in FIG. 2, with an auxiliary burner arrangement mounted on the shell.

FIG. 4 is a cross-section taken along line IV-IV in FIG. 3.

FIG. 5 shows part A depicted in FIG. 2, an embodiment of the burner provided with two rows of auxiliary nozzles.

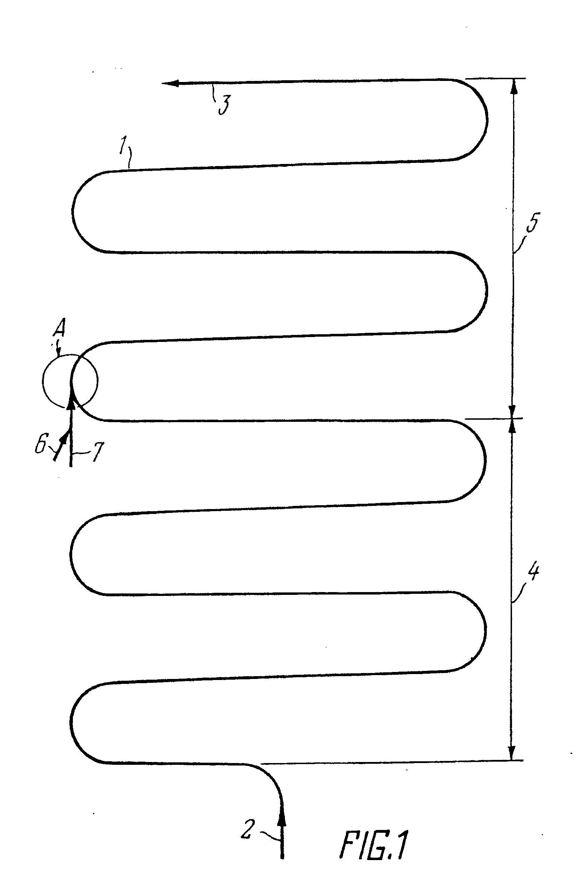

The apparatus according to the invention, shown in FIG. 1, comprises a shell 1 which is essentially a cylindrical coiled pipe provided with means 2 and 3 for feeding a hydrocarbon starting feedstock and for discharging of pyrolysis products, respectively. The shell 1 contains located consecutively therein along the flow of a the feedstock being processed a heating zone 4 and a pyrolysis zone 5. At the entrance of the pyrolysis zone 5 the shell is provided with by pipelines 6 and 7 made therein for feeding fuel and an oxidizer, respectively. The apparatus has adjustable heaters adapted for maintaining specified temperatures in the heating zone 4 and the pyrolysis zone 5 (for the sake of clarity said adjustable heaters are not depicted in FIG. 1).

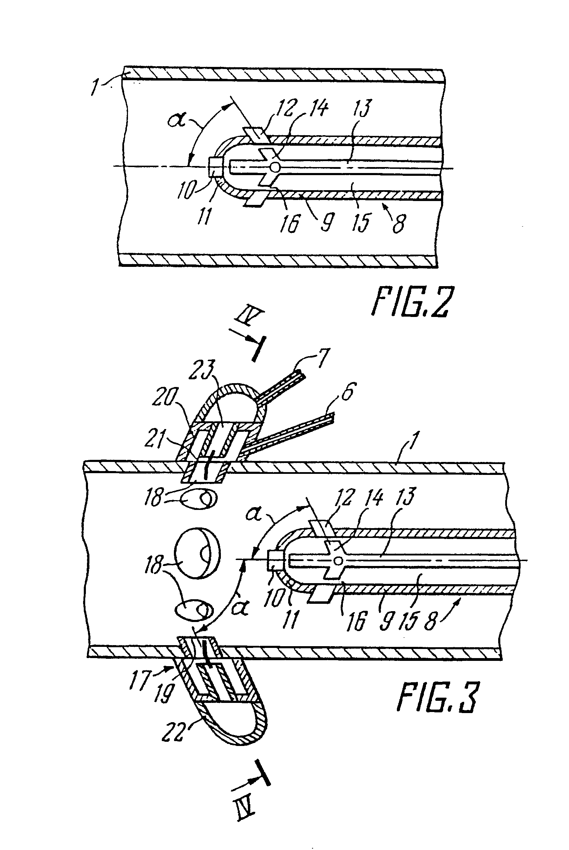

Mounted coaxially in the shell 1 is a burner 8 (FIG.2) having a cylindrical casing 9 which is provided with a nozzle 10 arranged coaxially therewith at an end-face 11 thereof, and with auxiliary nozzles 12 equidistantly spaced along the periphery of the casing 9 in one row. The auxiliary nozzles 12 of the burner 8 are directed at an angle $\alpha$ with respect to the longitudal axis of the shell 1, said angle $\alpha$ not exceeding $90°$. A central conduit 13 for feeding an oxidizer is coaxially mounted in the cylindrical casing 9, said conduit being provided with branches 14 equal in number to the number of the auxiliary nozzles 12. The conduit 13 and the inner wall of the casing 9 of the burner 8 define an annular passage 15 adapted to feed a fuel to the nozzle 10. Outlets 16 of the branches 14 open into the passage 15, each branch 14 being arranged coaxially to the respective nozzle 12. The other end of the burner 8 is fixed to the wall of the shell 1; the conduit 13 is connected to the pipeline 7 and the passage 15 is connected with the pipeline 6 (not depicted in FIG. 2). The burner 8 is located in a part A of the pyrolysis zone 5 (FIG. 1) where the temperature is maintained in the range of from 600 to $650°$ C.

Another embodiment of the present invention is shown in FIG. 3, which embodiment features, mounted on the outer side of the shell wall, a burner arrangement 17 which has nozzles 18 arranged in at least one row fixed in said wall in the vicinity of the burner 8 and uniformly spaced along its periphery. The nozzles 18 are located at the entrance of the pyrolysis zone 5 in the part thereof where the temperature is in the range of from 600 to $650°$ C and are directed at an angle $\alpha$ with respect to the longitudinal axis of the shell 1, said angle being less than 90° and preferably, being 45°. Outlet ends 19 of the nozzles 18 protrude beyond the inner wall of the shell 1. Installed on the shell 1 is an annular manifold 20 adapted for feeding a fuel. The manifold 20 has U-shaped passage area, is rigidly fixed to the shell 1 with its legs and communicates with inlet openings 21 of the nozzles 18. On the outer surface of the manifold 20 another annular manifold 22 is fixed adapted for supplying an oxidizer, said manifold having the passage area in the form of a semicircle. The manifold 22 communicates with the inlet openings 21 of the nozzle 18 through pipe connections 23 passing through the fuel manifold 20. The manifolds 20 and 22 communicate respectively with the pipelines 6 and 7. The distance between the burner 8 and the nozzles 18 of the burner arrangement 17 is selected observing the stipulation according to which any interaction between flame torches generated by the nozzles 10 and 12 and the nozzles 18 should be precluded. This distance is variable depending upon the exact location of the burner arrangement 17 at the entrance of the pyrolysis zone 5, which location in turn is selected in accordance with the diameter of the shell 1 and flame torches dimensions.

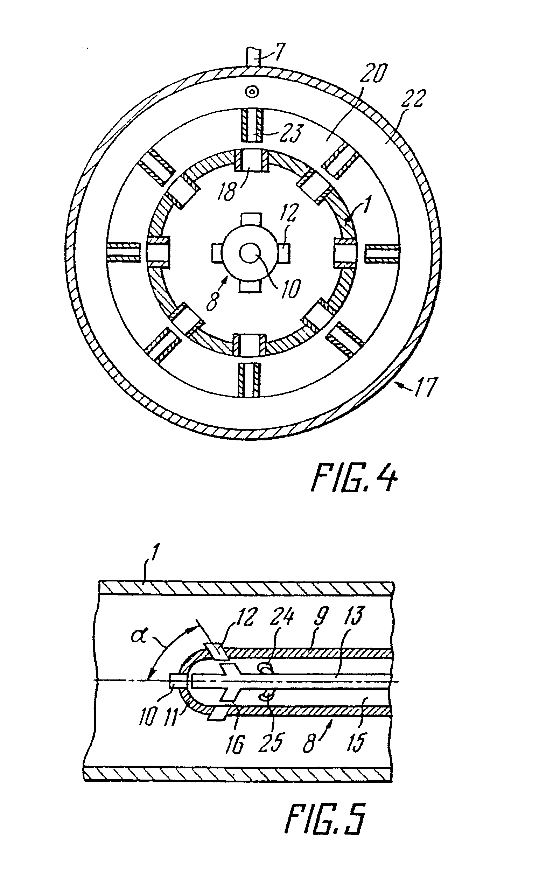

FIG. 4 shows a section along line IV-IV in FIG. 3 illustrating the apparatus cross-section with the burner arrangement 17 having eight nozzles 18 and eight pipe connections 23 and with the burner 8 having one nozzle 10 and four auxiliary nozzles 12.

FIG. 5 shows the same construction as that shown in FIG. 2 with the only difference that the burner 8 is equipped with the second row of nozzles 24 offset with respect to the nozzles 12 around the circumference and arranged in a staggered fashion. The conduit 13 is provided with the second row of branches 25 each of which is positioned coaxially to a respective nozzle 24 and has its outlet orifice opening into the passage 15 (not shown in Fig. 5).

A hydrocarbon feed stock which can be employed in the disclosed method includes light as well as heavy grades. Light grades of feedstock include naphtha, light gasoil, propane, pentane, ethane, and natural gas. Heavy grades of hydrocarbons which can be employed as a starting feedstock have flow temperature in the interval between -5°C and +150°C. Besides, heavy hydrocarbons obtained as by-products of purification processes can also be employed as a feedstock.

The method for producing lower olefines according to the invention is carried into effect in an apparatus shown in FIGs 1, 3, and 4. A flow of a hydrocarbon starting feedstock consisting of 55.7% by mass of propane and 43.3% by mass of butane and a flow of a diluent steam fed into the zone 4 of the shell 1 by means of the feeding means 2 with the rates respectively of 1,200 and 1,000 m³/h. The gaseous mixture is heated in the, zone 4 up to the temperature of 600°C.

A flow of oxigen is simultaneously fed at a rate of 60 m³/h into the burner 8 by the conduit 13 communicating with the pipeline 7 and with four branches 14, while by the passage 15 communicating with the pipeline 6 a flow of hydrogen is fed preliminary heated up to the temperature of 650 to 1,000°C and at a rate of 160 m³/h. Oxygen is fed into the burner arrangement 17 from the pipeline 7 by the manifold 22 and eight pipe connections 23 and hydrogen is fed from the pipeline 6 by the manifold 20. Flame torches of hydrogen in oxygen combustion are generated in the nozzle 10, and four auxiliary nozzles 12 of the burner 8 and in eight nozzles 18 of the burner arrangement 17. The nozzles 12 and 18 are arranged at an angle $\alpha$ equal to 45° with respect to the longitudinal axis of the shell 1.

Oxygen and hydrogen are fed into the burner 8 and the burner arrangement 17 in the volume ratio 1:4 respectively said ratio providing for complete reaction of hydrogen and oxygen in the flame torch thus precluding oxygen penetration into a hydrocarbon feedstock and formation of carbon oxides.

The indicated number of the nozzles 12 and 18 and their proper positioning in the zone 5 of the pyrolysis provides an integrated flame front along the axis and the periphery of the hydrocarbon feedstock flow bringing about an expansion of the interaction surface of the initiating additives formed in the flame torches, and hence, an increase in the yield of lower olefines. The time of interaction of the initiating additives and the hydrocarbon feedstock is about 7 ms.

The effect of the flame torces upon the process of pyrolysis consists in combined action of the radicals generated in the flames on the hydrocarbon feedstock simultaneously with a thermal overheating thereof in the torch zone. Therefore the high temperature of the hydrogen-oxygen flame also contributes to the effect of initiation.

The hydrocarbon feedstock already initiated is further pyrolysed in the pyrolysis zone 5 at a temperature about 800°C and during a contact time of 0.3 s. The resulting gaseous products of the pyrolysis are discharged from the pyrolysis zone 5 through the discharging means 3, abruptly cooled and separated by methods and installations known in the art.

Pyrolysis products contained, % by mass: $CH$-26.7%, $C_2H_6$-2.6%, $C_2H_4$-52.5%, $C_3H_8$-1.27%, $C_3H_6$-12.1%, $C_4H_8$-3.8%. The total amount of olefines equals 64.6% by mass.

Given in Table 1 are other embodiments of the disclosed method in the apparatus shown in FIGs 1, 3 and 4, as well as the process conditions and products yields.

Table 1

| | Heating temperature of feed-stock, °C | Pyroly-sis tempe-rature, °C | Interac-tion time of initia-ting addi-ves with feedstock, ms | Pyroly-sis contact time period, s | $H_2:O_2$ consump-tion rate per a litre of feedstock, l | ratio of $H_2$ to $O_2$, by vo-lume |
|---|---|---|---|---|---|---|
| 1 | 2 | 3 | 4 | 5 | 6 | 7 |
| I | 200 | 800 | 7,0 | 0,3 | 0,I:0,025 | 4:I |
| 2 | 300 | 800 | 7,0 | 0,3 | 0,I:0,025 | 4:I |
| 3 | 400 | 800 | 7,0 | 0,3 | 0,I:0,025 | 4:I |
| 4 | 500 | 800 | 7,0 | 0,3 | 0,I:0,025 | 4:I |
| 5 | 600 | 800 | 7,0 | 0,3 | 0,I:0,025 | 4:I |
| 6 | 650 | 800 | 7,0 | 0,3 | 0,I:0,025 | 4:I |
| 7 | 800 | 800 | 7,0 | 0,3 | 0,I:0,025 | 4:I |
| 8 | 600 | 600 | 7,0 | 0,3 | 0,I:0,025 | 4:I |
| 9 | 650 | 650 | 7,0 | 0,3 | 0,I:0,025 | 4:I |
| I0 | 650 | 700 | 7,0 | 0,3 | 0,I:0,025 | 4:I |
| II | 650 | 750 | 7,0 | 0,3 | 0,I:0,025 | 4:I |
| I2 | 650 | 800 | 7,0 | 0,3 | 0,I:0,025 | 4:I |
| I3 | 650 | 850 | 7,0 | 0,3 | 0,I:0,025 | 4:I |
| I4 | 650 | 920 | 7,0 | 0,3 | 0,I:0,025 | 4:I |
| I5 | 650 | 800 | 0,5 | 0,3 | 0,I:0,025 | 4:I |
| I6 | 650 | 800 | I,0 | 0,3 | 0,I:0,025 | 4:I |
| I7 | 650 | 800 | 4,0 | 0,3 | 0,I:0,025 | 4:I |
| I8 | 650 | 800 | 7,0 | 0,3 | 0,I:0,025 | 4:I |
| I9 | 650 | 800 | I0,0 | 0,3 | 0,I:0,025 | 4:I |
| 20 | 650 | 800 | I3,0 | 0,3 | 0,I:0,025 | 4:I |
| 2I | 650 | 800 | 20,0 | 0,3 | 0,I:0,025 | 4:I |
| 22 | 650 | 800 | 30,0 | 0,3 | 0,I:0,025 | 4:I |
| 23 | 650 | 800 | 40,0 | 0,3 | 0,I:0,025 | 4:I |
| 24 | 650 | 800 | 7,0 | 0,3 | I,24:0,3I | 4:I |
| 25 | 650 | 800 | 7,0 | 0,3 | I,00:0,25 | 4:I |
| 26 | 650 | 800 | 7,0 | 0,3 | 0,I6:0,04 | 4:I |

Table 1, continued

| I | 2 | 3 | 4 | 5 | 6 | 7 |
|---|---|---|---|---|---|---|
| 27 | 650 | 800 | 7,0 | 0,3 | 0,I44:0,036 | 4:I |
| 28 | 650 | 800 | 7,0 | 0,3 | 0,I2:0,03 | 4:I |
| 29 | 650 | 800 | 7,0 | 0,3 | 0,04:0,0I | 4:I |
| 30 | 650 | 800 | 7,0 | 0,3 | 0,I0:0,025 | 4:I |
| 3I | 650 | 800 | 7,0 | 0,3 | 0,036:0,00 9 | 4:I |
| 32 | 650 | 800 | 7,0 | 0,3 | 0,I:0,025 | I:I I,I:I,I |
| 33 | 650 | 800 | 7,0 | 0,3 | 0,I:0,025 | 2:I I,0:0,5 |
| 34 | 650 | 800 | 7,0 | 0,3 | 0,I:0,025 | 4:I I,0:0,25 |
| 35 | 650 | 800 | 7,0 | 0,3 | 0,I:0,025 | 6:I 0,6:0,I |
| 36 | 650 | 800 | 7,0 | 0,3 | 0,I:0,025 | 8:I 0,8:0,I |
| 37 | 650 | 800 | 7,0 | 0,3 | 0,I:0,025 | I0:I I,0:0,I |
| 38 | 650 | 800 | 7,0 | 0,3 | 0,I:0,025 | I2:I I,2:0,I |
| 39 | 650 | I050 | 7,0 | 0,0I | 0,I:0,025 | 4:I |
| 40 | 650 | I050 | 7,0 | 0,05 | 0,I:0,025 | 4:I |
| 4I | 650 | 900 | 7,0 | 0,20 | 0,I:0,025 | 4:I |
| 42 | 650 | 800 | 7,0 | 0,30 | 0,I:0,025 | 4:I |
| 43 | 650 | 700 | 7,0 | I,3 | 0,I:0,025 | 4:I |
| 44 | 650 | 650 | 7,0 | I,5 | 0,I:0,025 | 4:I |
| 45 | 600 | 600 | 7,0 | 2,0 | 0,I:0,025 | 4:I |

Table 1, continued

| | Pyrolysis Products Yield % by mass | | | | | | | |
|---|---|---|---|---|---|---|---|---|
| | $CH_4$ | $C_2H_6$ | $C_2H_4$ | $C_3H_8$ | $C_3H_6$ | $C_4H_{10}$ | $C_4H_{10}$ | total of olefines |
| 1 | 8 | 9 | 10 | 11 | 12 | 13 | 14 | 15 |
| I | I9,I4 | 7,5I | 39,9 | 0,7I | I3,I | 22,3 | 2,4 | 53,0 |
| 2 | 20,7I | I,72 | 4I,4 | 0,78 | I2,9 | I7,8 | 2,7 | 54,3 |
| 3 | 2I,50 | I,87 | 45,2 | 0,87 | I2,7 | I3,5 | 2,9 | 57,9 |
| 4 | 23,I3 | 2,0I | 48,7 | 0,99 | I2,6 | 8,8 | 3,I | 6I,3 |
| 5 | 24,30 | 2,3 | 5I,3 | I,I0 | I2,5 | 4,2 | 3,5 | 63,8 |
| 6 | 26,7 | 2,5 | 52,5 | I,30 | I2,4 | – | 3,9 | 64,9 |
| 7 | 33,5 | 2,5 | 49,2 | 0,98 | 9,I0 | – | 2,7 | 58,I |
| 8 | 4,I | 0,92 | 8,90 | 0,09 | 4,8 | 80,9 | 0,I5 | I3,7 |
| 9 | 5,2 | I,20 | I0,7 | 0,I0 | 5,0 | 77,I | 0,2I | I5,7 |
| I0 | I5,7 | 2,I5 | 30,9 | 0,29 | II,8 | 37,I | I,83 | 42,7 |
| II | 23,8 | 2,40 | 39,9 | 0,82 | I3,0 | I7,5 | 2,7I | 52,9 |
| I2 | 26,7 | 2,50 | 52,5 | I,3 | I2,4 | – | 3,9 | 64,9 |
| I3 | 30,7 | 2,30 | 50,I | I,I | II,2 | – | 3,5 | 6I,3 |
| I4 | 35,5 | 2,I | 48,9 | 0,8 | 8,4 | – | 2,8 | 57,3 |
| I5 | 2I,I | I,88 | 46,3 | 0,87 | I2,0 | I4,5 | 2,9 | 58,3 |
| I6 | 24,3 | I,90 | 50,2 | I,I0 | I2,9 | 6,I0 | 3,0 | 63,I |
| I7 | 25,9 | 2,30 | 52,I | I,2 | I2,5 | 2,6 | 3,6 | 63,6 |
| I8 | 26,6 | 2,7 | 52,5 | I,2 | I2,4 | – | 3,8 | 64,9 |
| I9 | 26,7 | 2,7 | 52,4 | I,I | I2,3 | – | 3,8 | 64,7 |
| 20 | 28,6 | 2,6 | 52,2 | I,3 | I0,7 | – | 3,8 | 62,9 |
| 2I | 28,9 | 2,6 | 52,0 | I,3 | I0,I | – | 3,7 | 62,I |
| 22 | 29,7 | 2,5 | 5I,8 | I,2 | 9,7 | – | 3,7 | 6I,5 |
| 23 | 32,6 | 2,4 | 50,8 | I,I | 7,5 | – | 3,6 | 58,3 |
| 24 | 29,8 | 2,5 | 5I,8 | I,I | 9,8 | – | 3,6 | 6I,6 |
| 25 | I8,I | 2,2 | 53,2 | I,2 | I0,9 | – | 3,5 | 64,I |
| 26 | 28,3 | 2,3 | 53,I | I,2 | II,4 | – | 3,5 | 64,5 |
| 27 | 27,9 | 2,4 | 53,0 | I,2 | I2,0 | – | 3,5 | 65,0 |
| 28 | 27,6 | 2,5 | 52,8 | I,2 | I2,3 | – | 3,6 | 65,I |

Table 1, continued

| I | 8 | 9 | IO | II | I2 | I3 | I4 | I5 |
|---|---|---|---|---|---|---|---|---|
| 29 | 27,8 | 2,5 | 52,6 | I,2 | I2,3 | – | 3,7 | 64,9 |
| 30 | 26,6 | 2,7 | 52,5 | I,2 | I2,4 | – | 3,8 | 64,6 |
| 3I | 30,I | 2,3 | 49,7 | I,I | I2,5 | – | 3,5 | 62,2 |
| 32 | 22,4 | 2,3 | 47,3 | I,2 | 9,7 | I3,I | 3,5 | 57,0 |
| 33 | 26,I | 2,7 | 52,4 | I,5 | I2,0 | – | 3,6 | 64,4 |
| 34 | 26,4 | 2,7 | 52,5 | I,4 | I2,4 | – | 3,8 | 64,6 |
| 35 | 26,2 | 2,6 | 52,3 | I,4 | I2,4 | – | 3,7 | 64,4 |
| 36 | 27,6 | 2,5 | 52,I | I,3 | I2,3 | – | 3,0 | 64,4 |
| 37 | 27,7 | 2,5 | 52,0 | I,3 | I2,2 | – | 3,6 | 64,2 |
| 38 | 28,7 | 2,6 | 5I,0 | I,4 | I2,2 | – | 3,7 | 63,2 |
| 39 | 23,4 | I,9 | 38,4 | 0,6 | II,8 | 22,5 | I,4 | 50,2 |
| 40 | 27,7 | 2,3 | 46,3 | 0,8 | I2,I | 7,8 | 2,6 | 58,4 |
| 4I | 29,I | 2,I | 5I,9 | 0,9 | I2,6 | 0,7 | 2,7 | 64,5 |
| 42 | 26,7 | 2,5 | 52,5 | I,3 | I2,4 | – | 3,9 | 64,9 |
| 43 | I8,2 | 2,4 | 33,4 | 0,7 | I0,3 | 30,0 | 2,9 | 43,7 |
| 44 | I2,4 | I,7 | I9,I | 0,8 | 8,7 | 52,3 | 2,2 | 27,8 |
| 45 | 2,2 | 0,3 | 2,8 | 0,I | 3,4 | 93,0 | 0,5 | 6,2 |

It is seen from the Table 1 that the maximum effect is obtained when the initiating additives intermediate products of combustion of hydrogen in oxygen - are fed to the feedstock at a temperature of from 600 to 650°C. The maximum value of the total yield of olefines and ethylene is observed at the pyrolysis temperature of 800°C and at the time of inter-reaction of the initiating additives and the hydrocarbon feedstock being 7 ms. The ratio of hydrogen and oxygen for the formation of the torch being within the range of 0.06 to 1.1 for hydrogen and within the range of 0.005 to 1.1 for oxygen, as calculated per litre of the feed stock has no effect on the yields of olefines.

The most acceptable ratio of hydrogen and oxygen supplied to the burner 8 and the burner arrangement 17 is 4:1 which makes it possible to obtain a high yield of ethylene and fully eliminate penetration of oxygen into the hydrocarbon feedstock and formation of carbon oxides.

Therefore, the results presented above show that the initiation of pyrolysis of the hydrocarbon feedstock with the products of burning the hydrogen-oxygen flame being in contact therewith under the above-specified conditions results in an increase of the yield of olefines by 5 to 10% by mass and a reduction of the pyrolysis temperature by 60 to 110°C and is accompanied by less power consumption of the process and a reduced thermal stress of the apparatus.

Example 46

The method according to the invention is carried into effect in the apparatus shown in FIGs 1 and 2. A hydrocarbon feedstock containing 55.7% by mass of propane and 43.3% by mass of butane and a diluent steam are fed into the heating zone 4 of the shell 1 by means of the feeding means 2 at a rate of 1,200 and 1,000 m³ respectively. A feedstock-diluent steam mixture is preheated in the heating zone 4 up to a temperature of 600°C. Fed simultaneously into the burner 8 through the conduit 13 communicating with the pipeline 7 is oxygen at a rate of 60 $m^3$/h, whereas fed through the passage 15 communicating with the pipeline 6 at a rate of 60 $m^3$/h is a flow of hydrogen preheated to from 650 to 1,000°C. Torches of hydrogen-oxygen flame are generated in the nozzle 10 and four nozzles 12 merging along the axis of the feedstock flow into the integnated flame front. The nozzles 12 are located at an angle about 35° with respect to the longitudinal axis of the shell 1. Oxygen and hydrogen are fed into the burner 8 in the ratio of their volumes 1:6 respectively. The time of the inter-reaction of the initiating additives and the hydrocarbon feedstock is 10 ms. The initiated feedstock is further passed to the pyrolysis zone 5 where it is subjected to the pyrolysis process at a temperature of 750°C, the contact time amounting to 1.0 s.

The gaseous products of pyrolysis are discharged through the means 3 and subjected to abrupt cooling and separation in facilities known in the art.

Pyrolysis products contain, % by mass: $CH_2$ - 25.4%, $C_2H_6$ - 3.04%, $C_2H_4$ - 50.6%, $C_3H_8$ - 3.1%, $C_3H_6$ - 11.4%, $C_4H_{10}$ - 0.7%, $C_4H_8$ - 4.7%. The total content of olefines amounts to 62% by mass.

Example 47

The method according to the invention is carried into effect in the apparatus shown in FIGs 1, 3 and 4; the difference resides in that at the initiation stage only the burner arrangement 17 is actuated. The process conditions are exactly like those in Example 29 of Table 1.

Flame torches generated in the nozzles 18 merge into an integral flame front over the periphery of a hydrocarbon feedstock flow. Resulting products of the pyrolysis process contained, % by mass: $C_2H_2$ - 25.9%, $C_2H_6$ - 2.9%, $C_2H_4$-51.3%, $C_3H_8$ - 2.1%, $C_3H_6$ - 12.2%, $C_4H_{10}$ 0.3%, $C_4H_8$ - 4.1%. The total content of olefines amounts to 63.5% by mass.

Example 48

The method according to the invention is carried into effect in the apparatus illustrated in FIGs 1 and 5.

A flow of a liquid hydrocarbon feedstock-hexane and a diluent steam are fed into the heating zone 4 of a the shell 1 through the feeding means 2 at the rates of 600 and 1,600 $m^3$/h, respectively. The mixture is heated in the heating zone 4 to a temperature of 600°C. Simultaneously oxygen is fed at a rate of 50 $m^3$/h into the burner 8 through the conduit 13 having branches 14 and 25 and communicating with the pipeline 7, and a flow of hydrogen preheated to a temperature of from 750 to 1,00°C at a rate of 160 $m^3$/h is fed through the passage 15 communicating with the pipeline 6. Torches of oxygen-hydrogen flame are generated in the nozzle 10, four nozzles 12 and four nozzles 24, the latter forming with the nozzles 12 a staggered row as on a chess-board. The nozzles 12 and 24 are arranged at the angle $\alpha$ of 60° with respect to the longitudinal axis of the shell 1.

The flame torches form a flame front along the axis of the hydrocarbon feedstock flow. The initiated feedstock is fed into the pyrolysis zone 5 where it is pyrolysed at a temperature of 700 to 800°C during the contact time period of 0.3 s.

The resulting pyrolysis products are removed through the means 3, cooled and separated in facilities known in the art.

Presented hereinbelow in Table 2 are the yields of olefines vs. the temperature of pyrolysis and, for the purpose of comparison, the yiels of olefines in a thermal pyrolysis carried out under the same conditions but without introduction of initiating additives.

Table 2

| Pyrolysis conditions | Pyrolysis temperature,°C | Products of pyrolysis,% by mass | | | | | |
|---|---|---|---|---|---|---|---|
| | | $CH_4$ | $C_2H_6$ | $C_2H_4$ | $C_3H_8$ | $C_3H_6$ | alkene |
| 1 | 2 | 3 | 4 | 5 | 6 | 7 | 8 |
| 1. With initiating additives | 700 | 13.7 | 3.18 | 38.0 | 0.5 | 18.8 | 0.9 |
| | 750 | 18.1 | 2.9 | 51.9 | 0.4 | 12.5 | 0.31 |
| | 800 | 22.8 | 3.0 | 59.0 | 0.27 | 8.5 | 1.1 |
| 2. Thermal pyrolysis | 700 | 9.13 | 2.7 | 28.3 | 0.5 | 16.4 | 0.1 |
| | 750 | 14.2 | 3.0 | 40.4 | 0.5 | 15.5 | 0.3 |
| | 800 | 16.6 | 2.8 | 48.4 | 0.3 | 11.7 | 0.5 |

Table 2,continued

| Pyrolysis conditions | Pyrolysis temperature,°C | Products of pyrolysis, % by mass | | | | Gas formation coefficient | |
|---|---|---|---|---|---|---|---|
| | | butene | divinyl | hexane | benzene | total of olefines | |
| 1 | 2 | 9 | 10 | 11 | 12 | 13 | 14 |
| I | 700 | 4.1 | 2.3 | 25.5 | 0.4 | 56.8 | 71.48 |
| | 750 | 1.4 | 2.7 | 6.58 | 1.56 | 64.4 | 89.21 |
| | 800 | 1.0 | 2.5 | 1.2 | 1.9 | 67.7 | 95.67 |
| 2 | 700 | 5.1 | 1.6 | 34.0 | 0.1 | 44.7 | 57.23 |
| | 750 | 4.3 | 2.7 | 16.2 | 1.1 | 55.9 | 80.9 |
| | 800 | 2.9 | 2.9 | 10.5 | 1.5 | 60.1 | 83.92 |

It is seen from Table 2 that introduction of initiating additives - intermediate products of combustion of hydrogen and oxygen in the process of hexane pyrolysis increases the yield of ethylene by 10% to 11% by mass as compared with the process of thermal pyrolysis, while the total yield of olefines is increased by 7% to 11% by mass.

# EP 0 451 280 A1

Table 3

| Yield of olefines, % by mass | Temperature in the zone of pyrolysis, °C | | | | | | | |
|---|---|---|---|---|---|---|---|---|
| | 550 | 600 | 650 | 700 | 750 | 800 | 850 | 920 |
| Process of the Invention | | | | | | | | |
| $C_2H_4$ | 3.2 | 8.9 | 10.7 | 30.9 | 39.9 | 52.5 | 50.1 | 48.9 |
| $C_3H_6$ | 1.6 | 4.8 | 5.0 | 11.8 | 13.0 | 12.4 | 11.2 | 8.4 |
| Process according to SU, A, 1268527 | | | | | | | | |
| $C_2H_4$ | 1.9 | 5.2 | 7.2 | 20.1 | 24.1 | 33.5 | 39.8 | 47.2 |
| $C_3H_6$ | 0.3 | 4.1 | 4.3 | 10.2 | 12.6 | 13.4 | 14.3 | 10.1 |

Table 3 shows the yield of olefines vs. the temperature of pyrolysis according to the disclosed process carried out in the apparatus shown in FIGs 1 and 2 and disclosed in SU, A, 1268558.

The comparative Table shows that the use of intermediate products of combustion of hydrogen and oxygen for the purpose of initiation is far more effective than the use of intermediate products of burning of hydrocarbon flames, since the rate of origination of the chains as well as completion of the process of pyrolysis are increased. Thus, the process of pyrolysis of the starting feedstock in the first case is practically completed at a temperature of 800° C while the maximum yield of ethylene in this case exceeds the yield of ethylene in the known process by 5% by mass at a temperature which is 120° C lower.

It should be clear to those skilled in the art that when the pyrolysis temperature is increased, the contact period should be decreased, and that the examples given in the specification are purely illustrative and do not limit the essence and the scope of the invention.

The present invention can be used advantageously in petrochemical industry.

## Claims

1. A method for producing lower olefines including the steps of heating a hydrocarbon feedstock, pyrolysis of the feedstock in the presence of initiating additives - intermediate products of burning of a fuel and an oxidizer, cooling of the products of pyrolysis formed and subsequent separation the end product therefrom, characterized in that used as the initiating additives are intermediate products of burning of hydrogen as the fuel and oxygen as the oxidizer, formed in the flame torch and introduced along the axis and/or periphery of the hydrocarbon, feedstock flow in the zone of pyrolysis by way of arranging therein at least one additional flame torch, and ensuring the time period of inter-reaction of the initiating additives and the hydrocarbon feedstock of 1 to 30 ms and a temperature of 600 to 650° C, the pyrolysis process being conducted at a temperature within the range of 600 to 800° C.

2. A method according to claim 1 characterized in that the pyrolysis process is effected within the time period of 0.05 to 1.5 seconds.

3. A method according to claim 1 characterized in that the formation of the intermediate products of burning is effected in the flame torch generated by supplying a flow of oxygen and, along the periphery thereof, of a parallel flow of hydrogen.

4. A method according to claim 3 characterized in that for the formation of the flame torch supplied are 0.1 to 1.0 l of hydrogen and 0.01 to 0.5 l of oxigen per one litre of the hydrocarbon feedstock.

5. A method according to claim 4 characterized in that a volume ratio of oxygen to hydrogen is 1:2-10.

6. An apparatus for producing lower olefines for carrying out the method according to claim 1 comprising a shell (1) provided with a means (2) for supplying of a hydrocarbon starting feedstock and a means (3) for discharging pyrolysis products and having located consecutively therein along the feed stock flow direction, a heating zone (4) and a pyrolysis zone (5), and a burner (8) installed in the shell (1) coaxially thereto and having a cylindrical casing (9) with a nozzle (10) located coaxially thereto at an end-face 11 thereof, and a central conduit (13) arranged in the cylindrical casing (9) and adapted for feeding an oxidizing agent, said conduit defining with the inner wall of the casing (9) of the burner (8) an annular passage (15) for feeding a fuel into the nozzle (10) characterized in that the burner (8) is positioned in the pyrolysis zone (5), particularly in the portion thereof where the temperature is in the range of from 600 to 650°C, and includes at least one row of auxiliary nozzles (12) equidistantly spaced around the periphery of its casing (9), the central conduit (13) being provided with a number or branches (14) corresponding to the number of the auxiliary nozzles (12) whose outlets (16) communicate with the fuel feeding passage (15), each of said branches being positioned coaxially with the respective nozzle (12).

7. An apparatus as claimed in claim 6 characterized in that it further includes a burner arrangement (17) mounted on the outer wall of the shell (1) and comprising at least one row of nozzles (18) fixed in the wall of the shell (1) in the vicinity of the burner (8) and equidistantly spaced along the perimeter thereof, whose outlet ends (19) protrude beyond the inner wall of the shell 1, an annular manifold (20) for feeding a fuel fixed to the shell (1) and communicating with inlets (21) of the nozzles (18), and another annular manifold (22) for feeding an oxidizer mounted on the outer wall of the first annular manifold (20) and communicating with the inlets (21) of the nozzles (18) by pipe connections (23) passing through the first manifold (20).

8. An apparatus according to claim 7 characterized in that a distance between the burner (8) and the nozzles (18) of the burner arrangement (17) is so selected as to preclude any interaction between the flame torches of the nozzles (12) and (18) of the burner (8), and of the nozzles of the burner arrangement (17).

9. An apparatus according to claim 7 characterized in that the auxiliary nozzles (12) of the burner (8) and the nozzles (18) of the burner arrangement (17) are positioned with respect to the longitudinal axis of the shell (1) at an angle less than 90°.

10. An apparatus according to claim 7 characterized in that the burner (8) is provided with two rows of the auxiliary nozzles (12) and the burner arrangement (17), with two rows of the nozzles (18), the nozzles of the preceding and suceeding rows being offset with respect to each other around the circumference and are arranged in a staggered order.

14

FIG.1

*FIG.2*

*FIG.3*

16

FIG.4

FIG.5

# INTERNATIONAL SEARCH REPORT

## I. CLASSIFICATION OF SUBJECT MATTER (If several classification symbols apply, indicate all) 6

According to International Patent Classification (IPC) or to both National Classification and IPC

Int.Cl.$^5$ C 07 C 4/04. C 10 G 9/00, 9/20 9/38

## II. FIELDS SEARCHED

### Minimum Documentation Searched 7

| Classification System i | Classification Symbols |
|---|---|
| Int.Cl.$^5$ | C 07 C 4/04; C 10 G 9/00, 9/20. 9/38 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | SU. A1. 1268558 (Kazakhsky gosudarstvenny universitet im. S.M. Kirova), 7 November 1986 (07.11.86) | 1-10 |
| A | US, A. 4527002 (MITSUBISHI JUKOGYO KABUSHIKI KAISHA), 2 July 1985 (02.07.85) | 1-5 |
| A | US. A. 4256565 (ROCKWELL INTERNATIONAL CORPORATION). 17 March 1981 (17.03.81) (cited in the description) | 1-5 |
| A | EP, A1. (NAPHTACHIMIE S.A.), 13 January 1988 (13.01.88) | 6-10 |

----------

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 30 October 1990 (30.10.90) | 12 November 1990 (12.11.90) |
| International Searching Authority | Signature of Authorized Officer |
| ISA/SU | |

Form PCT/ISA/210 (second sheet) (January 1985)